# EUROPEAN PATENT APPLICATION

(11) **EP 4 089 683 A1**
(43) Date of publication of application: **16.11.2022**
(21) Application number: 21382433.7
(22) Date of filing: 11.05.2021
(51) Int. Cl.: G16H 10/20, G16H 40/67, G16H 50/20, G16H 50/30

(54) **CONVERSATIONAL DECISION SUPPORT SYSTEM FOR TRIGGERING HEALTH ALARMS BASED ON WEARABLE DEVICES INFORMATION**

(71) Applicant: Barkibu, S.L., 15679 Cambre (ES)
(72) Inventor: PAZOS REY, Pablo, 15679 Cambre (ES)
(74) Representative: Tribalyte Ideas

(57) **Abstract**

The present invention provides a conversational decision support system for providing health care recommendations to a user and generating health related alarms, comprising: a server (2) which comprises information-storing means (4) storing health related information, and processing means (5) which in turn comprise a question set selection module (7) and an artificial intelligence, Al, based model (8); and a client application device (1) operated by a user for sending a health related query to the server (2). In response to said query, an exchange of questions and answers between the server (2) and the client application device (1) takes place. The system comprises an artificial intelligence-based model (8) trained with incomplete information. The question set selection module (7) selects the questions by considering the information introduced in the client application device (1) and the measurements registered by one or more wearable sensors (6). The invention further comprises a method for operating said system.

## Description

### FIELD OF THE INVENTION

The invention belongs to the field of telehealth services, and more particularly, veterinary health services. The system of the invention comprises a symptom checker and urgency detection functionality, in a conversational approach. Moreover, it combines information obtained with different wearable sensors and the answers provided by the pet owner, in order to make a pet assessment. Moreover, the system architecture is also suitable for its use in remote human assistance (i.e., in the case of babies or people with disabilities), being intended for the evaluation of a living being, who cannot provide to the system an accurate feedback or a proper description of its symptoms.

### BACKGROUND OF THE INVENTION

Conventionally, when a pet behaves different than normal or exhibits worrying symptoms, the owner's first option is to bring the animal to a veterinary emergency clinic. However, thanks to the development of technologies (particularly, mobile devices and applications), the field of remote health assistance for animals (pets) has evolved and nowadays there are several companies working in virtual veterinary systems, which enable the animal owner to perform a preliminary health assessment of the pet before bringing the animal to a veterinary clinic.

One of the companies working in a virtual veterinarian service are Vets4pets, which has developed a system ("Pet symptom checker") comprising a symptom checker module, urgency detection (triage based on the severity of the animal condition), suggestions of possible health conditions affecting the pet. This decision support system is conversational; thus, it requires an exchange of information between the pet owner and the system which is implemented as a software module for use on a mobile or desktop device. Another example is the company Sunrise Pet Clinic, which has developed a tool ("Pet Health checker") to provide information for deciding whether the pet's symptoms require veterinary attention or not, and how quickly that attention is needed. To get started, the system asks if the pet is a dog or cat (only these two are included), and then it offers a list of possible symptoms to choose. In this case, and similarly to Vets4pets, this system provides a symptom checker, urgency detection functionality, but it also includes symptom information and test recommendations.

Furthermore, there are also similar technologies oriented to remote human health assessment. This field is very active in the recent years, as demonstrated by the active research carried out by ITU focus group supported by the World Health Organization (WHO). Right now, the ITU focus group is trying to evolve the way of doing symptom assessment but is still in an earlier stage. Other companies (Ada Health and Babylon Health) apply artificial intelligence (Al) to similar systems providing human health recommendations.

There are also some companies working with conversational bot systems with a predefined question set to detect specific conditions. For example, patent application US 2018/0144099 A1 discloses a telehealth system with a predefined question set to detect common conditions. More specifically, it describes a decision support system to provide health care information oriented to animals. Said system comprises a client application device which downloads questions sets from a server, being these the answers to these questions determinant to diagnose the health of a pet. The server stores databases and animal data, along with the questions set and a catalogue of information. However, this system only provides general indications based on the information inputted by the pet's owner, without obtaining a personalized assessment for said pet. Moreover, the algorithm disclosed in US 2018/0144099 A1 is based on rules set by an expert a priori, such that the flow of questions that are displayed to the user are predetermined and cannot be changed.

Other known alternative in the state of the art is patent application US 2015/0066520 A1, which discloses a method for standardizing pet health care decision making, characterized in that it comprises a web/mobile application to enable a user inputting animal related queries, and an automatic system based on a decision tree. A key feature of this system is that, because of standardizing, each user providing the same input information is taken along the same branch of the tree, thus reaching the same diagnosis result. However, this point is a severe limitation of the system as it does not provide customized questions for a particular animal (depending on its particular physical condition). The method disclosed in patent application US 2015/0066520 A1 involves data queries that can be presented in sequence to the user (e.g., a pet guardian) so that the next questions adapt to the previous information provided by the pet guardian. This procedure with interlinked questions continues until the system has gathered enough information and outputs a health recommendation.

However, there exists a need for improved decision support systems for addressing pet or human health queries, providing a more personalized approach. Another problem in the case of pet health assessment is that the information available in the literature is scarce in comparison with human-related information, which hinders training the system. For this reason, training a decision support system oriented to pet health assessment is a challenging task.

Finally, the remote health systems (either oriented human or animal) typically require that a user fills information about the symptoms. However, sometimes the living being to be assessed (human or animal) cannot provide to the system an accurate feedback or a proper description of the symptoms (e.g., a baby or intellectually disabled individual), then it is not always possible to build a decision support system based on symptoms.

All the aforementioned problems are addressed by the system and method proposed by this invention.

### BRIEF DESCRIPTION OF THE INVENTION

The system of the invention is particularly intended for the evaluation of a living being (person or pet) who cannot provide an accurate symptom description or feedback through computer means. Rather, it is more convenient to use an approach based on observations rather than using symptoms as an input information. Consequently, the system would have an extra complexity as it must translate a set of observations into symptoms to feed the system, as later detailed.

The main object of the invention refers to a conversational decision support system for triggering alarms based on the information obtained by wearable devices. This system is conversational; thus, it requires an exchange of information (questions and answers) between a server, a client application device and wearable sensors. This system is different to other different existing decision support systems (either oriented to human or animal treatment) for obtaining alarms based on health recommendations because the user who answers the questions (e.g., a pet's guardian or the parent of a new born) is not the person or animal who has the possible health problem. In this case, the user who answers the question needs to interpret (give an observation) of the problem and try to explain it to the system. Our system allows them translating the problem they are perceiving into symptoms, such that the system generate an alarm in case of a serious health problem.

It is worth underlining that, unlike other known conversational systems in the field, the questions and answers evolve and are subjected to a learning process. In this way, instead of using a predetermined question and answers flow (e.g., for a certain answer to a question, the next question is always the same), in this invention the decision of the next question to be prompted in the client application device evolves depending on the input information and previous answers to questions.

In a first inventive aspect, the invention comprises a conversational decision support system for providing animal or human health care recommendations to a user and generating health related alarms, comprising:
- A server which comprises information-storing means storing animal or human related information and a plurality of questions, and processing means which in turn comprise a question set selection module and an artificial intelligence (Al) based model.
- A client application device operated by a user for sending a query to the server which comprises instructions stored on a non-transitory computer readable medium configured to:
   a) Present an initial application form to the user to collect input information about the query.
   b) Receive input information provided by the user on the initial application form.
   c) Send to the server the initial information included in the initial application form.
   d) Request, from the question set selection module of the server, a question set related to the animal or human condition.
   e) Display one or more questions from the question set in the client application, each question having one or more associated answers with a corresponding risk score.
   f) Receive selections of answers associated with at least one of the displayed questions.
   g) Send to the server the answers provided by the user.
   h) Repeat the three previous steps (e-g) to exchange question sets and answers between the client application device and the server until a health care recommendation based on the answers selected by the user is received from the server.
   i) Display the health care recommendation to the user in the client application.

Advantageously, the system is characterized in that it contains the following features:
- The question set selection module is configured to perform a next question algorithm to decide the most informative next question to be prompted to the user of the client application.
- The information storing means of the invention comprise a database with a plurality of symptoms organized hierarchically along with the corresponding health conditions associated with said symptoms.
- The Al-based model receives the question set from the question set selection module and the answers provided by the user through the client application. Then, the model outputs a health care recommendation and, optionally, an alarm is generated if the aggregated risk score of all the answers exceeds a predetermined risk threshold.
- The system further comprises at least one wearable sensor worn by the human or animal configured to collect biometric data and to fill the initial application form.
- The Al-based model is trained with incomplete information. In this way, we train the Al-based models with incomplete information, such that at least a part of the symptoms is unknown. This is a crucial feature of the system in order to make it less prone to missing input data or erroneous information provided by the user.
- The question set selection module selects the next question set by considering the information filled in the initial application form, the measurements registered by the at least one wearable sensor and the answers to the previous question sets, if any. The question set selection module further comprises a subsymptom basic module which is adapted to sort and update the symptoms stored in the information storing means, thereby updating the hierarchical relationships between different symptoms stored in the information-storing means. The information storing means, as later explained, is updated and learns with each new case that the system analyzes.

In some advantageous embodiments of the system, the question set selection module further selects the next question set by considering data which is stored in the information-storing means regarding previous uses of the system by the user for the same animal or human.

In additional embodiments of the system, the wearable sensors comprise at least one of the following: collar, ear tag, halter, belt, tag, or adhesive patch which are connected to the client application device and/or the server wirelessly. In this way, the system collects input data not only general pet information (like age, gender, breed, spaying, etc), but also wearable sensor data of the animal or human (amount of movement, heart rate, breath rate, etc.).

In certain preferred embodiments of the invention, the Al-based model is a deep neural network (DNN).

In some embodiments of the invention, the server and the client application device can be integrated in the same physical device (e.g., a conventional laptop, a tablet or a smartphone). In other embodiments, the server is a dedicated computer device, comprising hardware and software means to store and process the information collected by the client application device and the one or more wearable sensors, along with communication means to exchange information with the client application device and/or the wearable sensors through a communication network. Alternatively, the server can comprise cloud resources.

A second object of the invention refers to a method for obtaining an animal or human related health care recommendations along with possible alarm generation depending on said recommendations. Said method is performed by using the system described above to perform the following exchange of information between the server and the client application:
- Presenting in a client application device an initial application form to feed the system with an animal or human profile comprising a plurality of observations about the health condition of said animal or human.
- Collecting the initial information received in the initial application form and measurements acquired by, at least, one wearable sensor. Note that the chosen question set depends on two variables that makes this process fully personalized: the general pet information provided by the user of the client application device and the wearable sensor data. Consequently, in each case a user of the invention can be directed to a different flow of questions and answers, depending on wearable measurements or particular features of the animal or human being.
- Optionally, collecting initial information stored in the information-storing means regarding previous uses of the system for the same animal or human.
- Sending to a server the initial information.
- Requesting, from the question set selection module of the server, a question set related to the observations.
- Displaying one or more questions from the question set to the client application, the question having one or more answers and each answer being associated with a corresponding risk score.
- Sending to the server the answers associated with the displayed questions.
- Selecting the next question set to be sent to the client application device depending to the answers to the previous question set.
- Repeating the four previous steps until a health care recommendation, based on the selected answers and the measurements registered with the wearable sensor, is received from the server.
- Displaying said health care recommendation to the user.
- Sending an alarm to the user if the risk score exceeds a predetermined risk threshold.

Advantageously, the method considers the information registered by wearable sensors (collar, etc.) to guide the process and to select the more appropriate next question. In this system, the system is able to dynamically choose the more informative question set (linked to the next most probable symptom), which enables a fast evaluation with the minimum number of questions but with a high degree of reliability. When the method considers that the information is enough to recommend a certain course of action (e.g., to schedule an appointment with a vet, to treat the pet at home or to generate an alarm)., then the Al-based model, e.g., a deep neural network, outputs the most probable conditions related to the case and an alarm indication (e.g., a boolean value indicating whether the pet requires immediate veterinary assistance or if home treatment could be appropriate). The decision about whether enough information has been obtained to stop is made through an algorithm that takes into account different variables: number of questions and answers already treated, quality of the questions that remain unanswered, etc. In each exchange of information (exchange of a question and an answer), a reliability parameter is computed and, when said parameter reaches a safety threshold, the method may stop. The system also enables urgency detection by considering the frequency and duration of the symptoms and the measurements from the wearable sensors for the risk score assessment.

In optional embodiments of the aforementioned method, each answer of the question set is associated with a risk score which updates an aggregated risk score with each observation and at least a subset of the questions of the question set and the corresponding answers provided by the user are used to detect the urgency of the animal or human condition. In even further embodiments of the method, said method is immediately terminated in any point of the execution when the aggregated risk score of all the answers exceeds a predetermined risk threshold, and wherein said termination is accompanied with an alarm generation. In those cases, the system sends to the user a warning message (or an alarm) recommending immediate attention. In those cases, the system enables establishing a communication with a veterinarian or clinician and providing him/her with all the information gathered by the system.

In particular embodiments of the invention method, the question set is determined by the question set selection module from the answers to the initial application form which define an initial profile (of the pet or human, with general information) and a first observation, the measurements registered with at least one wearable sensor and, optionally, from the database information stored in the information-storing means about previous uses of the system for the same animal or human. Said method comprising the following steps (also referred to as recommendation and alarm generation job):
- Associating the first observation, the initial profile and the measurements of the at least one wearable sensor with an existing symptom in the knowledge database. In this way, the observations are translated into symptoms. It is worth pointing out that this system works with observations, not with symptoms. In this way, this novel layer is applied to turn the observations into medical symptoms, for feeding the system.
- Finding the next most probable symptom in the knowledge database by performing the following substeps:
   a) Splitting the list of symptoms asked to the user into present, not present or unknown, each symptom being associated with a risk score.
   b) Filtering symptoms by getting all cases that have the same initial profile and getting all cases that have all symptoms marked by the user and/or the wearable sensor (6) as present; and then removing all cases that have one or more symptoms marked by the user as not present.
   c) Grouping symptoms (by key) in filtered cases and sum their aggregated risk score.
   d) Sorting symptoms by aggregated risk score in descending order.
   e) Selecting the first symptom, associated with the highest risk score, as the next question to be prompted to the user.
- Repeating the substeps a) to e) with new observations provided by the user until no symptoms matching filtering criteria, a predefined number of questions is reached, or if a termination condition and alarm generation is received.
- Finally, generating the alarm, if needed (when the alarm generation condition, based on exceeding a maximum risk score value, is reached).

In certain embodiments, multiple urgency thresholds/levels are defined to provide a more accurate triage depending on the observations inputted through the client application device.

In the previous method the questions have been prompted to the user of the client application device sequentially, one in each exchange of information with the server. Other embodiments of the invention may involve the exchange of multiple questions and answers in each communication with the server.

In certain embodiments, the step of associating the first observation with an existing symptom in the database further comprises the use of the information stored in a database of the information-storing means about previous uses of the system for the same animal or human. In this way, information collected in previous records can be considered to simplify the exchange of information between the server and the client application.

In further preferred embodiments of the method of the invention, it previously comprises the training of the Al-based model of the system by following these steps:
a) Providing information about a plurality of cases, each one comprising symptoms and the corresponding health assessments, i.e., stored in information-storing means, said means comprising a knowledge database.
b) Processing said information by applying data augmentation and extract, load and transform techniques to create two datasets.
c) Generating a training set by considering a part of the first dataset.
d) Adding noise to the training set.
e) Defining a plurality of Al-based models with a different hyperparameter (e.g., number of neuron layers) configuration. Advantageously, every time a model needs to be updated, a plurality of different models is trained (the so-called grid of models) rather than training just a single model. In this way, different combinations (permutations) of hyperparameters are applied and the resulting models are trained.
f) Testing the Al-based models of step e) with the validation set which comprises the remaining part of the first dataset which is not used in step c). The goal is, by making use of each of the trained Al-based models, predicting the condition corresponding to the symptoms of each case in the validation set. In this way, after the test one obtains a prediction error which characterizes the accuracy of the model. From all the models of the grid, the one providing the best performance (lower prediction error) is chosen.
g) Selecting the Al-based model providing the lower prediction error in the first dataset.
h) Using the Al-based model obtained in step g) as an initial model and repeating the steps c) to g) by using the second dataset to refine the previous estimation of the model.
i) Outputting the model obtained in step h) as the final Al-based model to perform the method described above.

According to the previous method, obtaining the optimum model requires a supervised approach and cross-validation. However, other embodiments of the method may involve different training methods.

The system of the invention described above overcomes different technical problems of the known solutions in the field.

As it has been outlined in the background section, one technical problem present in the field is how to provide a more personalized decision support system which takes into account particular physiological condition of a person or pet. To overcome this problem, the system of the invention further comprises the input of heterogeneous information from hardware sensors, combining it with the questionary information, in order to select the more appropriate questions to diagnose the pet or person and to guide the final user depending on the result. Additionally, the system selects the following question that must be sent to the user considering the observations and the sensor (particularly, wearable devices worn by the person o pet to be assessed) measurements. In this way, the assessment is fully personalized, such that the sensor measurements can significantly shorten the question and answers exchange with the system. For instance, depending on the measurements registered by a sensor (e.g., a wearable collar), some questions shall be omitted, or inconsistent information inputted by the pet guardian can be amended, which leads to a more reliable assessment. The wearable sensor measurements serve to detect physiological data along with behavioural patterns by processing said physiological data. In this way, the sensor information has a synergic effect with regard to the health assessment and alarm generation, as the exchange of questions and answers between the client application device and the server is regulated, at least in part, by the information collected by the wearable sensors.

The second technical problem, the limited data available for training such a system. Training data with theoretical symptoms are useful as the system requires many observations to properly train the DNN.

The third technical issue is that the system is intended to obtain information about pet animals or people unable to properly describe their symptoms, so it must be adapted to overcome erroneous symptoms and redirect the final user towards a correct health evaluation regardless certain amount of incorrect input information. For this reason, training the Al-based model of the system with incomplete information is a crucial feature. With each new real case that the system analyzes, it learns, and it is able to update the set of questions and answers such that the resulting question set is the most informative with the minimum number of questions. Consequently, the question set can be updated.

### BRIEF DESCRIPTION OF THE DRAWINGS

To complete the description and in order to provide for a better understanding of the invention, a set of drawings is provided. Said drawings form an integral part of the description and illustrate an embodiment of the invention, which should not be interpreted as restricting the scope of the invention, but just as an example of how the invention can be carried out. The drawings comprise the following figures:
Figure 1 shows a preferred embodiment of the system of the invention, comprising the server, the client application device and at least one wearable sensor which collects information of a pet.
Figure 2 illustrates the workflow followed by the system from the initial information gathering until the decision to issue an alarm or not.
Figure 3 shows a schematic representation of the should stop algorithm.
Figure 4 shows a possible implementation of the deep neural network of the artificial intelligence-based model.

In these figures, the following reference numbers are used:

| | |
|---|---|
| 1 | Client application |
| 2 | Server |
| 3 | Initial application form |
| 4 | Information-storing means |
| 5 | Processing means |
| 6 | Wearable sensors |
| 7 | Question set selection module |
| 8 | Al-based model |

### DETAILED DESCRIPTION OF THE INVENTION

Here, we describe possible implementations of the system, which is the main element of the object of the invention. It is important to understand that following embodiments only intend to illustrate particular designs of such system.

The system on the invention relies on artificial intelligence (Al) for the creation of health assessment recommendations and alarms, either oriented to animals or humans. In particular, it is suitable for providing decision support in health assessment, as well as for the generation of warning messages or alarms depending on the severity of the health condition of a living being, for instance, a pet. Said system performs a conversational algorithm that asks the pet owner for information to detect if there could exist a health problem in its pet, such that there is an exchange of information (as questions and answers) between a client application device (1) operated by a user and a server (2) which comprises the machine-readable instructions to process said exchange of information. The system takes into account the information provided by the pet owner to decide which is the most informative question to ask, in order to obtain a fast health assessment of the pet with the lower number of questions. In this way, the system (which is also referred to as virtual veterinarian) works as an alarm tool such that it generates alarms depending on the clinical condition of the pet.

In subsequent paragraphs, the system is described only for its use in animal assessment (as a virtual veterinarian) and corresponding alarm generation. However, the system can be readily applied in humans (e.g., babies) with no communication capabilities for describing by themselves the symptoms they are undergoing.

Pet owners without knowledge of medicine cannot know if something is wrong with their pet without attending a veterinary clinic. In this way, the system of the invention helps pet owners to detect if the animal has some health problem, by triggering an alarm if a concerning symptom is detected. Nevertheless, the system is not based on symptoms but on observations made by a different user (e.g., the pet owner). In this way, considering that the animal cannot describe its symptoms properly, this system comprises an extra layer of complexity in comparison with other existing systems, in order to deal with incomplete (erroneous or missing) information provided by the pet guardian, instead of just feeding the system with the raw pet information provided by said guardian related to the pet profile. The pet profile comprises information like the age, breed, weight, neutered status, and other data (i.e., genetic information) obtained from existing medical record system or provided by the pet guardian. Optionally, if genetic data of the pet is available, then the system is more likely to detect the presence of a genetically related condition or discard that condition completely with a shorter set of questions and answers. Some examples of conditions wherein this genetic data is very useful for the system would be:
- Hip-Dysplasia, a typical hereditary condition in a lot of big dog breeds and can be discarded with a genetic test.
- Degenerative myelopathy. A progressive neurological condition caused by the deterioration of nerve fibers.
- Cataracts: the hereditary form of this disease shows marked breed specificity in ophthalmic appearance, age of onset and rate of progression of the condition.
- Pancreatitis: some breeds have a strong genetic predisposition to pancreatitis (e.g., in Miniature Schnauzers species).

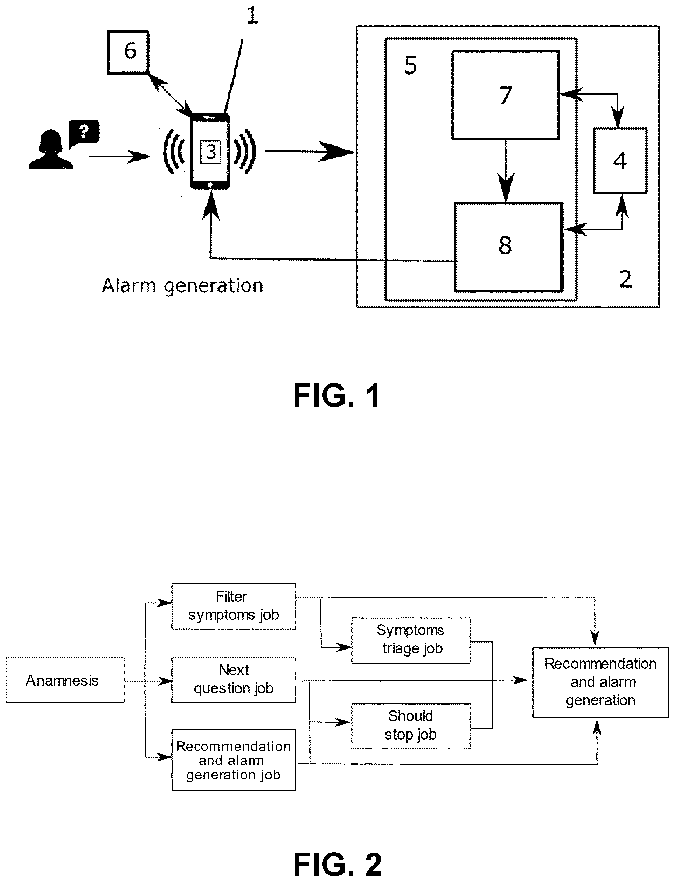

A preferred embodiment of the invention is summarized in Figure 1, which comprises a client application device (1), e.g., a smartphone or other smart device, which shows the user an initial application form (3), for inputting information about a health query. The input information in the system comprises, for instance, age, species, or even genetic information of the pet. Once the initial application form (3) is filled, the information is sent to a server (2) in which it is processed. The client application device (1) may download a series of interlinked questions and answers from the server (2) or may exchange said questions and answers with the server (2) through a network (e.g., Internet, or local area network). The server (2) comprises information-storing means (4) (e.g., a database) for storing the dataset of questions and possible answers associated with the health state, along with additional information (e.g., diseases or relevance of each symptom in the possible diseases). Moreover, the server (2) comprises processing means (5) adapted to receive and process the information provided by the user, and to send back appropriate information (e.g., next question to be asked to the user depending on the answer to, at least, one of the previous ones, possible diagnosis according to the known symptoms, alarms, etc.). Additionally, the system relies on one or more wearable sensors (6) to gather data regarding the pet behaviour and habits, such that it enables performing a personalized health recommendation and alarm generation process when the user detects a possible disease and uses the system (e.g., implemented in a mobile device) to check it. The information obtained by the wearable sensors (6) affects to the questions that are prompted to the user. The wearable sensors (6) are communicated with the server (2) and the client application device (1) through a communication network (e.g., Internet, radio, local area network, etc.).

Information about the pets can be stored in the information-storing means (4) for successive information queries by the same user and for the same pet. Particularly, genetics tests can be done to the pets in order to detect some predisposition to any condition. In this case, said information can be also included in the initial application form (3), stored in the information-storing means (4) before the user starts a consultation or even stored in one wearable sensor (6), so as to give personalized health recommendation or alarm depending on particular features (species, condition, genetic tests, etc.) of the pet.

In this way, the invention does not replace a real veterinarian, but it only tries to provide a tool suitable for getting a good first health assessment of the animal and providing alarms depending on the health condition of the pet, thus recommending the pet's guardian the next steps to take.

The health condition assessment, once the symptoms are gathered and the profile data is built, is similar same for humans and pets from a technical point of view. This means that hardware deployment (communication networks, use of wearable sensors (6), etc.) and metrics (threshold risks, etc.) applied in certain embodiments of this invention for generating the pet health alarms, validation of the symptoms, and so on are compatible with human health assessment.

However, this invention exhibits several differences in comparison with existing systems for remote health assessment and alarm generation. A major difference between human and pet assessment arises from the information gathering perspective, taking into account the limited feedback provided by the animal. Humans can give quite clear descriptions on how they feel, indicating the degree of pain or explaining the specific area in which it occurs. This also applies to indications about when the pain started, how it progressed, if it is an intermittent or constant and so on. On the other hand, this information is not available for animal assessment, thereby the information depends on another person inputting in the system the symptoms of an animal whose communication or feedback is limited. For this reason, the raw information that the system of the invention collects does not comprise symptoms but observations, unlike other decision support systems for remote health assessment which are just asking for symptoms to the user and feeding the system with them.

These observations are translated into symptoms when the processing means (5) of the system analyze them to assess if the observations are plausible or some missing or erroneous data exist in said information. That is to say, the system must translate and interpret the pet owner observations into medical symptoms, especially when these symptoms are vague. For instance, a common case example: a dog/cat who is lethargic, typically described as "he is not himself and not eating as much as usual" (so, the observations inputted to the system would be lethargic & decreased appetite). However, the pet could exhibit such symptoms because it suffers from a tummy ache, but it cannot confirm that, nor the pet parent can identify said clinical condition in most cases. Therefore, reaching a health-related recommendation is much more complex, so as to generate an alarm if the condition is serious or require immediate veterinary assistance. Obtain observations has a lot of implications to ease the accuracy of the alarms provided by the system, for instance, taking into account the time the pet owner spends with the animal, the time of day of these interactions and the number of pet owners involved (the walking human, the feeding human, the player human, etc.). All this information can be input in the initial feeding form (6).

Since the system user only can indicate one or more observations (rather than the real symptoms that the pet is undergoing), the use of wearable sensors (6) allows obtaining real information about what is happening to the pet, thereby helping the system to better understand the context and the particular health condition of the pet. In this way, this system has one extra layer of complexity to deal with erroneous input observations which may lead to miss an important symptom or accept in the assessment a non-important symptom. Consequently, the combination of information provided by the pet's owner (i.e., provided by filling the initial application form (3) or available in the information-storing means (4) of the system if it is not the first time the pet's owner uses the system) along with the data collected by the one or more wearable sensors (6) worn by the pet help to make a better health recommendation and generating health related alarms, if required.

Another relevant difference with existing human and pet health assessment decision support systems is that the information provided by the pet guardian might be erroneous or incomplete, thus hindering a proper health assessment. The fact that this system is able to work with partial or unknown data also affects the use of wearable sensor (6) information, as the system would give a more accurate health assessment and a more accurate alarm generation when said information is available, but it will be able to work even when some of this information is not accessible.

Another problem regarding pet health assessment is the scarce information available to train the system. Particularly, the processing means (5) comprises a question set selection module (7) and an Al-based model (8) which is responsible to associate observations to symptoms, and then translating said symptoms to a health recommendation (or diagnostic) along with the decision whether generations a possible alarm or not when a risk threshold is overcome. This Al-based model (8), which preferably comprises a deep neural network (DNN), requires information to be properly trained. However, in veterinary medicine the amount of peer-reviewed published data is limited, unlike human health data which is more easily found. It is interesting to underline that even the most popular textbooks differ in how they describe conditions in veterinary medicine. For this reason, the development of a complete database of clinical cases is needed, as there are no clear benchmarks suitable for this task. To address this problem, the present invention is specifically designed to overcome missing information by providing a customized real-case database to the system, and then completing it with synthetic data of theoretical cases that match the reality (data augmentation approach). Moreover, the system is trained under noisy conditions, as later explained, which makes it robust against missing or erroneous input information.

It is worth remarking the question set selection module (7) is configured to be updated with each real case that the Al-based model (8) analyzes, through a learning process, in order to obtain the most informative question set with the smaller exchange of information (minimum number of questions) between the server (2) and the client application device (1).

In this way, the Al-based model (8) is trained with a dataset stored in the information-storing means (4) and containing a combination of real and theoretical cases, which comprise relationships between the pet profile, symptoms and the corresponding diagnosed conditions, including:
a) Real cases that happen in a clinic.
b) Theoretical (synthetic) cases, which are created from the relations between symptoms and conditions added by expert vets in our knowledge database. These synthetic cases enlarge the database of cases meanwhile enough real cases are not feasible. The theoretical cases are treated to be uncomplete, since we need to train the Al-based model (8) such that it can reach the most likely conditions corresponding to a case even without having all the symptoms information. For this purpose, theoretical cases are created by randomized deletion and/or addition of one or more symptoms, before its use for training the Al-based model (8). In this regard, it is worth underlining that genetic data is optional to improve the Al-based model (8) accuracy, but it's not strictly needed.

Moreover, the system of the invention handles the situation where we do not have all the information regarding a case as two situations where we do not have all the symptoms are common:
- When the user does not understand the pet situation and is not able to translate the clinical condition to the alarm system.
- When during the answers-question process we do not ask about a symptom that the pet has, due to incomplete input information.

Additionally, various big data, statistical and machine learning techniques are applied to support missing or erroneous data due to the discrepancy between pet parent observations, clinical signs, and the real effects of the conditions in the pet. Those techniques include:
- Data augmentation. To complete the cases from the real-case database and using our knowledge base, permutations of theoretical cases are used.
- The generation of various degrees of noise in the training set to simulate the error in the user input due to the process of doing symptoms assessment on behalf of a third party is that the pet in this case.
- Train the Al-based model (8) with incomplete data, such that not all the symptoms are known. In this regard, a subsymptom basic module is also provided in the question set module (7), which alters the next question process performed by the question set selection module (7) and auto-completes the input data provided by the user, thereby avoiding errors or inconsistencies. The question set selection module (7) takes advantage of the organization of the symptoms following hierarchical relationships or dependences in the database of the information-storing means (4), e.g., a database wherein each symptom could have a parent and one or more childs. For instance, a symptom "bloody diarrhoea" is a daughter of the more general symptom "diarrhoea". Advantageously, this relationship enables shortening the exchange of questions and answers between the server (2) and the client application device (1). Particularly, the subsymptom basic module is configured to sort and update the symptoms stored in the information-storing means (4) following hierarchical relationships, thus avoiding that the question set selection module (7) could ask for a "parent" symptom once it is known that a "child" symptom occurs. For instance, if its already known that "haemoptysis" is a symptom, then the system does not require to ask whether the more general "cough" symptom is present. In this way, the exchange of information required to obtain a reliable health recommendation is simplified and possible redundancy in questions prompted in the client application device (1) is avoided.

Preferably, the database stored in the information-storing means (4) is organized in rows, each one representing a case, while each column corresponds to different symptoms (attributes for each case). For instance, said database contains a plurality of symptoms, age, different conditions (e.g., allergies), spaying status (binary value, 0 or 1), gender, etc.

Advantageously, the wearable sensor (6) can detect different observations, for instance, a lack of movement for a prolonged period, or a progressively reduced activity over a few days, especially at times when the pet usually moves according to its daily routine. These observations are translated into the following symptoms: lethargic, depressed, or exercise intolerance. The quantitative information of the wearable sensor (6) can be converted into behaviour patterns of a pet, i.e., turning in the bed or pacing at home. More particularly, if the system detects increased activity at times when there's usually no pet activity (e.g., during the night), the following considerations are considered by the Al-based model (8):
a) If the detected activity pattern is associated with the pet turning in its bed (observation), then the system considers that a symptom could be that the pet has painful joints or abdomen, such that it cannot find a comfortable position to rest.
b) If the detected activity pattern is associated with pacing around the house, the system considers that a possible symptom could be diarrhoea, need to urinate, excessively thirsty or disoriented, or even need to vomit. These possibilities are considered to decide the more informative next question that the pet owner would answer in the client application device (1).

The information provided by the wearable sensors (6) may also enable the finding of predisposing factors, for example:
- Decreased activity over time would predispose to obesity.
- Activity that is lower than average for that age and breed could also lead to obesity.
- Sudden, "violent" episodes of exercise in a 4-year-old Labrador who starts limping afterwards would make a cruciate ligament rupture differential diagnosis more likely.

By combining these predisposing factors with others that we already have from the pet's profile (including genetic information) as filled in the initial application form (3), or from previous interactions or assessments of the same pet with the system. For instance, if the Labrador of the previous example has obesity as a predisposing factor and It does not have the dysplasia gene mutation in his genetic test, then a diagnosis involving cruciate ligament injury is more likely. In this case, the system would generate an alarm with a recommendation of immediate bringing the dog to the veterinarian.

Moreover, the system may be provided with video data from Internet of Things (loT) devices (for instance, installed in the final user home) to obtain relevant pet attributes for the health recommendation.

Thanks to the wearable sensors (6) it is possible monitoring the condition of animal based on orientation, movement, and physiological data (heart rate, temperature, breathing rate, saliva contents, muscle contraction, movement or lack of it, different levels of intensity of this movement, tremors, convulsions, scratching) of an animal registered by a plurality of monitorization wearable sensors (6) like a collar, ear tag, halter, belt, tag or adhesive patch which may be connected to the client application device (1) and/or the server (2) wirelessly (e.g. radio or Internet-based communications). In the case of providing human health alarms, the wearable sensor (6) can be an activity sensor, chest band for heart monitoring, temperature sensor, etc.

In a preferred embodiment, the wearable sensors (6) comprise an activity tracking collar, which input valuable information in the system that the pet owner does not know (e.g., pet pacing at night). Moreover, wearable sensor (6) information could help to validate the observations given by the pet parent, thus confirming them if there is a match with one or more possible conditions, or if there is a mismatch if the information provided by the pet parent is unlikely or incompatible according to the known observations.

Optionally, other invention embodiments may also include wearable sensors (6) for acquiring image and video (e.g., digital camera or other loT devices) to monitor the pet. In those cases, images are also used to feed the system.

As shown in Figure 2, the workflow of the system includes different processes (jobs):
- Anamnesis, which implies gathering all the input data of the pet including observations. This step is done by the client application device (1), and the input information is sent to the server (2).
- By taking the input information, the processing means (5) of the server (2) performs the three different jobs: filtering symptoms (translating user observations into symptoms to feed the Al-based model (8)), next question process carried out by the question set selection module (7) and the recommendation and alarm generation job described below. The alarm generation job is carried out in the neural network.

- A should stop process, in charge of deciding when stopping the next question process.
- A symptoms triage process, depending on the severity and frequency of the symptoms.
- Presentation of more probable conditions (health recommendations) and possible alarm generation.

Below, the recommendation and alarm generation job is described.

When the user starts a consultation in the virtual veterinarian by using the client application device (1), and before starting the exchange of questions and answers, the preliminary information available (pet data) is collected to make the best personalized diagnosis flow. The pet data the system handles comes from several sources, as we previously mentioned, including the pet profile provided by the pet owner (including age, spaying status, gender, breed, etc.), data registered by the wearable sensors (6) which provides personalized question set according to the pet status, and genetic tests which enable the detection of predisposition to a series of conditions, thus contributing to a more personalized diagnosis process for the pets as the question set is governed by all this information. For instance, certain genetic markers can automatically discard the presence of certain diseases and adapt the next question to be prompted to the user. Other option could be to adapt the question set prompted to the user depending on the gender of the pet or the spaying status. At the same time, the order of priority given to some questions can be changed depending on previous answers to questions and all the input information available for the pet. Once all the input information is ready, the system works as described below.

Firstly, the system gets initial data (anamnesis) as populated in the initial application form (3) and a first observation. In this way, a pet profile is arranged, with at least one of the following data: species, age, sex, neutered status, breed, wearable(s) information; along with the first observation (comprising information of duration and frequency of said observation).

Secondly, more observations are obtained after a process of question-answer between the client application device (1) and the server (2), which takes place as follows:
- The system is provided with a dataset stored in the information-storing means (4) (a knowledge database) of the server (2), comprising pet profiles with symptoms and conditions.
- The user introduces a pet profile and a first observation through the client application device (1).
- With the first observation, the server (2) calculates the next question about a new observation. For example, this first observation is associated with an existing symptom in the knowledge database, obtaining a symptom key. This symptom can be searched by the symptom name or by related condition name.
- While the process does not end, the following steps are performed in a loop for the next observations:
   a) The server (2) asks for a new observation.
   b) The client application device (1) replies to the server (2) depending on said observation is present or not in the animal, or depending on whether the answer is unknown by the user.
   c) The server (2) receives the answer of the client application device (1) and translates the observation into a symptom.
   d) Depending on said symptom, a question set selection module (7) of the server (2) calculates which is the most informative next observation to ask the user of the system taking into account the previous observations, the information of the initial application form (3) and the analytical Al-based model (8). Optionally, information obtained in previous uses of the system for the same pet can be also considered.

The process for obtaining the most informative next question to be prompted to the user of the system is detailed below. The next question algorithm asks for the presence of the next most probable symptom in the dataset, which comprises the realization of the following steps:
a. Creating a list with all the possible symptoms and assigning them a relevance score (further referred to as weight or risk score) depending on the previously selected symptoms and the pet profile introduced in the system by means of the client application device (1), and also depending on the distribution of symptoms and conditions relationship in the information-storing means (4), being that relationship calculated by an algorithm that mixes expert knowledge and clinical data.
b. Splitting the list of symptoms asked to pet parent depending on whether they are present or not, or they are unknown.
c. Filtering the different symptom cases:
   i. Get all cases that have same pet profile of pet according to the information inputted by the user in the initial application form (3).
   ii. Get all cases that have all symptoms marked by pet parent as present.
   iii. Remove those cases that have some mandatory symptom indicated by pet parent as not present.
d. Grouping symptoms by key in filtered cases and summing its aggregated weight.
e. Remove those symptoms that have been already asked (either as present, not present or unknown).
f. Sort the symptoms by decreasing aggregated weight.
g. Take first one as next question to be prompted to the user in the client application device (1) or returning null if there are no symptoms that match filtered criteria in some step of this algorithm.

This iterative process for obtaining the next question is repeated until the finish is decided depending on possible conditions and urgency. The system decides when it has sufficient information to finish and provide a health recommendation and generate an alarm, if needed. Together with the finish condition, possible conditions with explanations and additional information related to the urgency of them is also delivered. In this way, the next question process is repeated until there are no more symptoms to ask for, if a predefined number of questions is reached, or if a stop command from the so-called "should stop algorithm" is received.

The so-called "should stop algorithm" is responsible for determining when the system has enough information such that the Al-based model (8) provides reliable results. Otherwise, the process continues until a reliable solution is reached. This algorithm uses the score (aggregated weight) associated with each condition in the output of the analytical model to decide if it has enough information to stop asking. When the aggregated weight of all the symptoms reaches a confidence threshold (a predetermined value), then the output of the system demonstrates enough confidence for the conditions presented and the algorithm stops. As a summary:
- If there is not next question, then should stop algorithm finish the iterations (returns a true value).
- If next symptom to be asked is only optional because the information provided to the system is enough, to reach the confidence threshold, then should stop is true and this is considered as a terminator.
- In any other cases, the should stop algorithm returns false and continues the iterative process of questions and answers.

At the end, at the finish point of the should stop algorithm, at least two symptoms must be found to provide a health recommendation. If not, the algorithm outputs a warning message explaining that no health recommendation can be delivered.

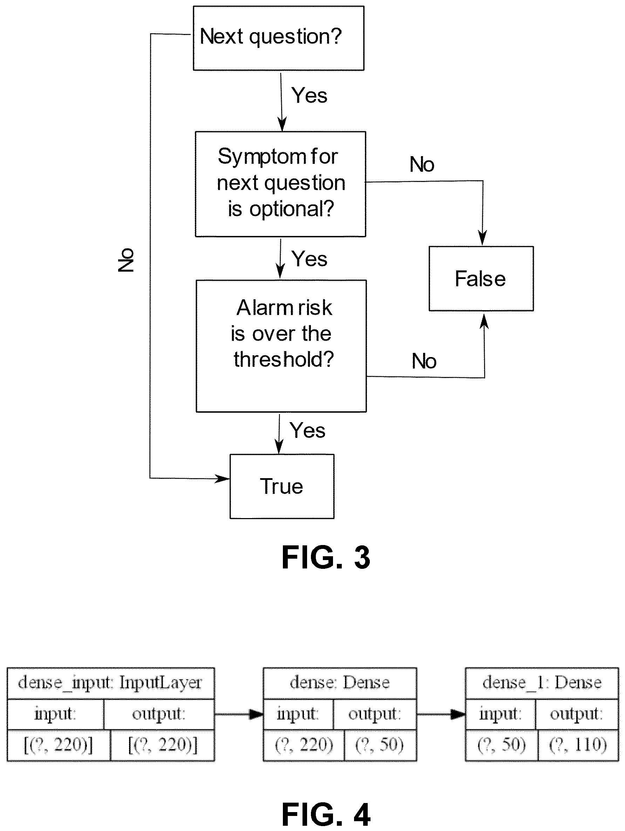

The should stop algorithm is illustrated in Figure 3, wherein the iterative questions and answers procedure continues until the system considers that it has sufficient information to output a health recommendation or a course of action. Otherwise, more information is requested to the user. Consequently, the system dynamically adapts the questions depending on the input information. If the system determines that there is a high-risk score (an emergency threshold is exceeded), a warning is sent to the pet owner, thereby recommending an immediate visit to the veterinarian.

Thirdly, and once we have all the input data (anamnesis) available, we need to adapt them into the required format by the Al-based model (8). For this reason, a module for adapting the data from heterogeneous information sources (the initial application form (3), the one or more wearable sensors (6), etc.) is needed. Once said adaptation has been carried out, all these data are used to feed the prediction Al-based model (8). This module is also responsible for filling incomplete data that are not available with default values representing the unknown status. Finally, all those values are converted into an array (i.e., numeric array format) that is used to feed the Al-based model (8), for instance, a neural network.

In this way, thanks to the consideration of individual information of the pet (i.e., obtained by wearable sensors (6)) apart from general information (obtained in the initial application form (3) and associated with the age, breed, species, etc.) the system allows us to create the best set of questions personalized for the pet so as to obtain the maximum amount of information with the lower number of questions, thus finding the most probable conditions for the case, as well as generating an alarm when required.

The system further comprises an urgency detection module, thus providing a triage functionality (e.g., low, medium or high urgency levels). For each symptom, the frequency and duration are asked. In the storing-information means (the database), we have a record of urgency for symptoms and conditions, as well as frequency and duration thresholds for said symptoms which may alter the order of the questions, the urgency of the possible treatment, and the generation of the alarm. Also, by means of the wearable sensors (6), we can detect urgent situations related to one or more parameters considered with the wearable (e.g., if an excessive heart rate is detected with a wearable, then an alarm is generated). The final urgency presented to the user in the client application device (1) is the higher urgency among the selected observations and the predicted conditions.

With regard to the Al-based model (8), the structure of said model is preferably a deep neural network that uses supervised training to predict the conditions from the data on our dataset.

The training process of the Al-based model (8) takes place following the next sequence of steps:
- Data augmentation process, comprising extract, transform and load (ETL) techniques are applied to generate the training dataset.
- Noise (i.e., random noise) is added to the training data.
- Multiple Al-based models (8) with different hyperparameter configurations are trained following a grid model.
- The Al-based models (8) are tested against the test data selecting the best one of them as the final Al-based model (8).
- The final Al-based model (8) structure used for the system is trained with a more complete dataset and validated with the validation set.

Finally, we recall that the system also comprises a database combining real clinical cases and theoretical cases, in order to enlarge the information available for training the deep neural network (supervised approach) of the Al-based model (8). This model (8) is trained using a combination of computer processing units (CPUs) and graphical processing units (GPUs), either local and/or cloud-based architecture.

A preferred embodiment of the Al-based model (8) of the system is included in Figure 4, which corresponds to a multilayer DNN, including an input layer, a set of hidden layers and an output layer. Each of these layers may have a different number of input and outputs, depending on the model (8) and its performance during the training process. For instance, the number of input neurons in the input layer depends on the considered number of symptoms and pet information (in this case, 220 inputs). In the output layer, the number of output neurons corresponds to the number of different conditions which can be detected with the system (in this case, 110 outputs). Additionally, the DNN comprises an additional output neuron which is associated with the alarm generation. In other embodiments, more hidden layers can be arranged in the DNN.

Finally, some general remarks about the invention. The next question algorithm implemented in the question set selection module (7) is not based on Al. Conversely, the prediction (e.g., a convenient health recommendation with a certain reliability parameter) obtained by the system is Al-based. The result of the prediction (e.g., when a reliability parameter is reached) could impinge on the point at which the system stops asking questions to the user, however, said prediction result does not alter the questions displayed to the user of the client application device (1).

In other embodiments of the invention, one or more wearable sensors (6) are able to detect anomalies in the measurements regarding a human or animal and emitting an alarm without requiring that user launches a query by means of the client application device (1). In that case, the process of questions and answers starts autonomously in the client application device (1), thereby collecting the information from the one or more wearable sensors (6) and previous information of the same human or pet available in the information-storing means (4) regarding previous uses of the system. In this way, the client application device (1) serves as an intermediate between the one or more wearable sensors (6) and the server (2), regulating the exchange of information between them.

Additional advantages and modifications will readily occur to those skilled in the art. Therefore, the invention in its broader aspects is not limited to the specific details and representative embodiments shown and described herein. Accordingly, various modifications may be made without departing from the spirit or scope of the general inventive concept as defined by the appended claims and their equivalents.

## Claims

1. A conversational decision support system for providing health care recommendations about a human or animal to a user and generating health related alarms depending on said recommendations, comprising:
- a server (2) comprising information-storing means (4), which stores health related information and a plurality of questions, and processing means (5) which in turn comprise a question set selection module (7) and an artificial intelligence, Al, based model (8);
- a client application device (1) operated by a user for sending a query about a human or animal health condition to the server (2), said client application device (1) connected to the server (2) through communication means and comprising instructions stored on a non-transitory computer readable medium configured to:
present an initial application form (3) to the user to collect input information about the query;
receive input information provided by the user on the initial application form (3);
send to the server (2) the initial information of the initial application form (3);
request, from the question set selection module (7) of the server (2), a question set related to the health condition;
display one or more questions from the question set in the client application device (1), each question comprising a request about a symptom and having one or more associated answers with a corresponding risk score;
receive answers associated with at least one of the displayed questions;
send to the server (2) the answers provided by the user;
repeat the three previous steps to exchange question sets and answers between the client application device (1) and the server (2) until a health care recommendation based on the answers selected by the user is received from the server (2); and
display the health care recommendation to the user in the client application device (1); and
said system being **characterized in that:**
the system further comprises at least one wearable sensor (6) worn by the human or animal, connected to the client application device (1) and the server (2) through communication means, and configured to collect biometric data in order to fill the initial application form (3);
the information storing means (4) store information associated to a plurality of symptoms organized hierarchically along with the corresponding health conditions associated with said symptoms;
the question set selection module (7) is adapted to select the most informative next question to be prompted to the user of the client application device (1) by considering the information filled in the initial application form (3), the measurements registered by the at least one wearable sensor (6) and the answers to the previous questions, if any;
the question set selection module (7) further comprises a subsymptom basic module which is adapted to sort and update the symptoms stored in the information storing means (4); and
the Al-based model (8) is adapted to receive the question set from the question set selection module (7) and the answers to said question set provided by the user through the client application device (1), and to output a health care recommendation and an alarm if the aggregated risk score of all the answers exceeds a predetermined risk threshold.

2. The system according to the preceding claim wherein the question set selection module (7) is adapted to further select the next question set by considering data which is stored in the information-storing means (4) regarding previous uses of the system by the user for the same animal or human.

3. The system according to any of the preceding claims, wherein the wearable sensor (6) comprises at least one of the following: collar, ear tag, halter, belt, tag or adhesive patch which are connected to the client application device (1) and/or the server (2) wirelessly.

4. The system according to any of the preceding claims, wherein the Al-based model (8) is a deep neural network.

5. A method for obtaining an animal or human related health care alarm, wherein said method comprises the use of the system of claims 1-4 to perform the following steps between the server (2) and the client application device (1):
presenting, in a client application device (1), an initial application form (3) to feed the system with an animal or human profile comprising a plurality of observations about the health condition of said animal or human;
collecting the initial information received in the initial application form (3) and measurements acquired by, at least, one wearable sensor (6);
sending to a server (2) the initial information;
requesting, from the question set selection module (7) of the server (2), a question set related to the observations;
displaying one or more questions from the question set to the client application device (1), the question having one or more answers and each answer being associated with a corresponding risk score;
sending to the server (2) the answers associated with the displayed questions;
selecting the next question set to be sent to the client application device (1) depending to the answers to the previous question set;
repeating the four previous steps until a health care recommendation, based on the selected answers and the measurements registered with the wearable sensor (6), is received from the server (2);
displaying said health care recommendation to the user; and
sending an alarm to the user if the risk score exceeds a predetermined risk threshold.

6. The method according to the preceding claim, wherein collecting the initial information further comprises collecting initial information stored in the information storing means (4) regarding previous uses of the system for the same animal or human.

7. The method according to the claims 5-6 wherein each answer to the question set is associated with a risk score which updates an aggregated risk score with each observation and at least a subset of the questions of the question set and the corresponding answers provided by the user are used to detect the urgency of the animal or human condition.

8. The method according to the preceding claim wherein said method is immediately terminated in any point of the execution when the aggregated risk score of all the answers exceeds a predetermined risk threshold, and wherein said termination is accompanied with an alarm generation.

9. The method according to any of the claims 5-8, wherein the question set is determined by the question set selection module (7) from the answers to the initial application form (3) which define an initial profile and a first observation, the measurements registered with at least one wearable sensor (6); the method comprising the following steps:
- associating the first observation, the initial profile and the measurements of the at least one wearable sensor (6) with an existing symptom in the database;
- finding the next most probable symptom in the database by performing the following substeps:
splitting the list of symptoms retrieved asked to the user into present, not present or unknown, each symptom being associated with a risk score;
filtering the list of symptoms by getting all cases that have the same initial profile and all symptoms marked by the user and/or the wearable sensor (6) as present while discarding all cases that have one or more symptoms marked by the user as not present;
grouping the list of symptoms in filtered cases and compute their aggregated risk score;
sorting the list of symptoms by aggregated risk score in descending order;
selecting the first symptom in the list, associated with the highest risk score, as the next question to be prompted to the user; and
- repeating the previous step with new observations provided by the user until no symptoms matching filtering criteria, a predefined number of questions is reached, or if a termination condition or alarm generation condition is received; and
- if the alarm generation condition is reached, generating the alarm.

10. The method according to the preceding claim, wherein the step of associating the first observation with an existing symptom in the database further comprises the use of the information stored in a database of the information-storing means (4) about previous uses of the system for the same animal or human.

11. The method according to any of the claims 5-10, wherein said method previously comprises the training of the Al-based model (8) of the system by following these steps:
providing information about a plurality of cases, each one comprising one or more symptoms and the corresponding health assessments, which are stored in information-storing means (4);
processing said information, by applying data augmentation and extract, load and transform techniques, to create a first dataset and a second dataset;
generating a training set by considering a part of the first dataset;
adding noise to the training set;
defining a plurality of Al-based models (8), and training each of said Al-based models (8) with a different hyperparameter configuration;
testing the models of the previous step with the cases included in the validation set which comprises the remaining part of the first dataset not used for generating the training set, in order to obtain a prediction error;
selecting the Al-based model (8) providing the lower prediction error in the first dataset;
using the Al-based model (8) obtained in the previous step as an initial Al-based model (8) and repeating the five previous steps with the second dataset;
outputting Al-based model (8) obtained in the previous step as the final Al-based model (8) to perform the steps of the method according to claims 5-10.
